# EUROPEAN PATENT APPLICATION

(11) **EP 4 101 495 A1**
(43) Date of publication of application: **14.12.2022**
(21) Application number: 21179117.3
(22) Date of filing: 11.06.2021
(51) Int. Cl.: A61N 1/05

(54) **RING ELECTRODE WITH A SPECIAL CONTACTING CHANNEL**

(71) Applicant: Heraeus Deutschland GmbH & Co. KG, 63450 Hanau (DE)
(72) Inventor: SINANOVIC, Erol, 63450 Hanau (DE); RICHTER, René, 63450 Hanau (DE)
(74) Representative: Glas, Holger

(57) **Abstract**

The present invention relates to a ring electrode for a medical device comprising a monolithic metal tube, wherein the monolithic metal tube has a wall which in one section comprises a contacting channel, wherein the contacting channel extends into the axial direction of the monolithic metal tube, and wherein the contacting channel makes at least a partial turn around the center axis of the monolithic metal tube. The invention further relates to a medical device comprising the ring electrode and to a process for preparing the ring electrode.

## Description

### FIELD OF THE INVENTION

The invention relates to a ring electrode for a medical device and to a medical device comprising a ring electrode. The invention also relates to a process for preparing a ring electrode.

### BACKGROUND OF THE INVENTION

Ring electrodes for medical devices such as cardiac pacemakers or neuromodulation devices are known in the art. Known ring electrodes often contain one or more contacting channels which are attached to the wall of the ring electrode for contacting a wire to the electrode. Due to manufacturing constraints the contacting channels of known ring electrodes usually extend parallel to the center axis of the ring electrode in axial direction. This means that the contacting channels of known ring electrodes are formed by a straight inner lumen which extends in the same direction as the wall of the ring electrode to which it is attached.

A wire which is to be contacted to the ring electrode is usually provided as part of a coil. Thus, the wire has to be unwound and bend out of its coiled or spiral form to be inserted into the straight contacting channel of the electrode. The bending of the wire leads to a weak spot in the wire, which can result in a lead fracture. Furthermore, the bending of the wire can lead to an insufficient contacting between the wire and the ring electrode and/or a decreased reliability for a welded contact between the wire and the electrode.

Moreover, certain lead assemblies known in the art contain ring electrodes and contacting channels, wherein the contacting channels are not integral parts of the ring electrode, but form part of a separate component. In such a case, the ring electrodes and the separate component comprising the contacting channel need to be assembled in one or several steps. This can lead to an increased risk of failure modes and can be complex and/or costly.

In view of the above, there is a need in the art for a ring electrode, which is suitable for use in a medical device, which allows for an improved contacting of a wire. It is further desired that the ring electrode is made from as little components as possible.

Therefore, the present invention is directed to an improved, or at least, alternative ring electrode. The present invention is further directed to a medical device comprising such a ring electrode and to a process for manufacturing such a ring electrode.

### SUMMARY OF THE INVENTION

In one aspect, the invention provides a ring electrode for a medical device. The ring electrode comprises a monolithic metal tube,
wherein the monolithic metal tube has a wall which in one section comprises a contacting channel,
wherein the contacting channel extends into the axial direction of the monolithic metal tube, and wherein the contacting channel makes at least a partial turn around the center axis of the monolithic metal tube.

The present invention is based on the finding that a contacting channel, which does not extend parallel to the tube of the ring electrode but turns at least partially around the center axis of the electrode, allows for an improved contacting of a coiled wire with the ring electrode. The contact between the wire in the ring electrode is improved, because the contacting channel is better aligned with the coiled or spiral form of the wire so that the wire does not need to be bend to a significant extent before being inserted into the contacting channel. Since the wire is not bend, the wire and/or the contacting channel is less mechanically strained. Thereby, lead fractures and/or weak contacts between the wire and the electrode are avoided. The present invention further comprises that the shape of the contacting channel can be varied based on the specific wire which is used. For example, if a coiled wire is used which has a low pitch angle, a contacting channel can be designed which also has a low pitch angle. In this way a bending of the wire can be avoided as much as possible.

Another advantage of the inventive ring electrode is that the contacting channel isolates the wire from its surroundings which reduces, or fully avoids, the risk of a short cut between the wire and e.g. another wire which is in communication with another electrode.

Moreover, the inventive ring electrode is based on a monolithic metal tube. This means that the ring electrode in its simplest form is a one-piece material, which does not need to prepared from two or more components.

Another aspect of the present invention is directed to a medical device comprising the ring electrode according to the present invention and a wire, wherein the ring electrode is contacted to the wire by its contacting channel.

Yet another aspect of the present invention is directed to a method for preparing the ring electrode according to the present invention, wherein the process comprises the step of preparing a monolithic metal tube by a metal additive manufacturing process.

It should be understood that for the purposes of the present invention, the following terms have the following meanings:
A "monolithic metal tube" in the meaning of the present invention is a metal tube which essentially consists of, preferably consists of, one material having a homogeneous structure without boundary lines and/or interfaces between different components.

A "contacting channel" in the meaning of the present invention is an inner lumen in the wall of the monolithic metal tube having a first end and a second end, wherein at least one end, and preferably both ends, have an opening for inserting a wire.

The "axial direction" of the ring electrode is the direction of the center axis of the ring electrode. The "center axis" of the ring electrode is the axis which intersects perpendicular with the center of a radial plane of the ring electrode.

"At least a partial turn" means that the contacting channel makes a turn of at least 5° around the center axis of the monolithic metal tube.

Where an indefinite or definite article is used when referring to a singular noun, e.g., "a", "an" or "the", this includes a plural of that noun unless anything else is specifically stated.

Where the term "comprising" is used in the present description and claims, it does not exclude other elements. For the purposes of the present invention, the terms "essentially consisting of" and "consisting of' are considered to be a preferred embodiments of the term "comprising". If hereinafter a group is defined to comprise at least a certain number of embodiments, this is also to be understood to disclose a group, which preferably essentially consists only of these embodiments, or preferably consists only of these embodiments. Furthermore, the term "essentially consisting of" includes the term "consisting of", which is to be understood as a preferred embodiment of the term "essentially consisting of".

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment. Whenever the terms "including" or "having" are used, these terms are meant to be equivalent to "comprising" as defined hereinabove.

### BRIEF DESCRIPTION OF THE DRAWINGS

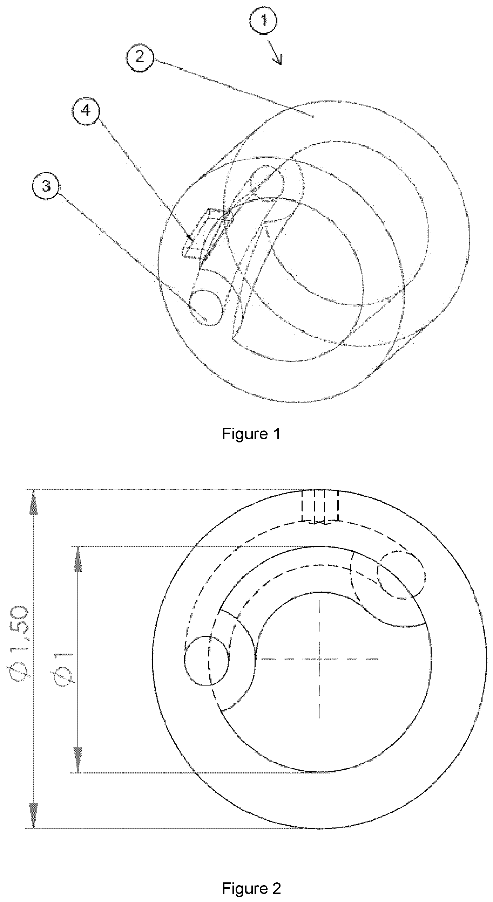

The following schematic drawing shows aspects of the invention for improving the understanding of the invention in connection with some exemplary illustrations, wherein
- Figure 1: shows schematic drawing of an inventive ring electrode 1 consisting of a monolithic metal tube 2. The monolithic metal tube 2 has a wall which in one section comprises the contacting channel 3. The contacting channel 3 extends into the axial direction of the monolithic metal tube 2, whereby the contacting channel 3 makes a partial turn around the center axis of the monolithic metal tube 3. The wall section with the contacting channel 3 protrudes into the inner cavity of the monolithic metal tube 2. The outer lateral surface of the monolithic metal tube 2 has an opening 4 which connects the outside to the contacting channel 3.
- Figure 2: shows a technical drawing of the ring electrode according to the example as seen in axial direction. The ring electrode has an inner diameter of 1 mm and an outer diameter of 1.5 mm. The contacting channel makes a partial turn around the center axis of the monolithic metal tube of about 125°.
- Figure 3: shows a technical drawing of the ring electrode according to the example as seen in a top view. The electrode has an opening which connects the outside to the contacting channel. The opening has a length of 0.40 mm and width of 0.03 mm.
- Figure 4: shows a technical drawing of the ring electrode according to the example as seen in a side view. The electrode has a length of 3 mm.
- Figure 5: shows a cross section along the C-C line in Figure 4.
- Figure 6: shows a magnification of the encircled part D of Figure 5.
- Figure 7: shows a black/white image of two ring electrodes prepared on the basis of technical drawings 2 to 6.
- Figure 8: shows a helix with a pitch P and a center axis C.
- Figure 9: shows a helix with a pitch angle α between a plane PL, which is perpendicular to the center axis C, and a tangent T of the helix.

### DETAILED DESCRIPTION

One aspect of the present invention is directed to a ring electrode for a medical device. The ring electrode comprises a monolithic metal tube, wherein the monolithic metal tube has a wall which in one section comprises a contacting channel, wherein the contacting channel extends into the axial direction of the monolithic metal tube, and wherein the contacting channel makes at least a partial turn around the center axis of the monolithic metal tube.

The ring electrode according to the invention comprises a monolithic metal tube.

The ring electrode may consist of the monolithic metal tube. In other words, the ring electrode may be the monolithic metal tube.

However, it is also possible that the ring electrode comprises a monolithic metal tube and a coating layer, which is present on the monolithic metal tube. A "coating layer" in the meaning of the present invention is a thin layer of material, preferably in the range of 0.1 to below 10 µm. Suitable coatings are metal nitrides such as TiN, metal oxides such as IrO₂ or conductive polymers.

The monolithic metal tube is preferably a biocompatible metal. The term "biocompatible" in the meaning of the present invention is meant to refer to a material which is considered by a person skilled in the art to be safe when being in contact with a living organism (e.g. a human) over a specific period of time (e.g. when used in an implantable medical device). A skilled person knows or can determine whether a metal can be considered biocompatible or not. For example, the biocompatible metal may be a biocompatible metal according to EN ISO 10993.

According to a preferred embodiment, the monolithic metal tube is a metal selected from the group consisting of iron, silver, copper, nickel, palladium, platinum, gold, iridium, steel, titanium, hafnium, niobium, tantalum, cobalt, chromium, zirconium, rhenium, tungsten, molybdenum, and alloys of each one of these metals. More preferably, the metal is selected from the group consisting of nickel cobalt alloy, stainless steel, platinum and platinum alloys. Most preferably, the metal is a platinum iridium alloy.

A suitable stainless steel is e.g. stainless steel AISI 316 L. A suitable nickel cobalt alloy is MP35N^{®}. MP35N^{®} is a nickel cobalt alloy comprising nickel, cobalt, chromium, and molybdenum. Suitable platinum iridium alloys are Pt/lr 10 or Pt/lr 20. Most preferably, the platinum iridium alloy is Pt/Ir 10.

The monolithic metal tube can be defined by an outer diameter and an inner diameter of the inner cavity of the monolithic metal tube.

According to one embodiment, the monolithic metal tube has an outer diameter in the range of 0.2 to 3.0 mm, preferably in the range of 0.3 to 2.5 mm, more preferably in the range of 0.5 to 2.0 mm. Preferably, the outer diameter is a constant outer diameter.

According to one embodiment, the monolithic metal tube has an inner diameter in the range of 0.1 to 2.9 mm preferably in the range of 0.2 to 2.4 mm, more preferably in the range of 0.3 to 1.9 mm. The "inner diameter of the monolithic metal tube" refers to the one inner diameter in the monolithic metal tube which is the largest inner diameter.

According to a preferred embodiment, the monolithic metal tube has an outer diameter in the range of 0.2 to 3.0 mm, preferably 0.5 to 2.0 mm, and an inner diameter in the range of 0.1 to 2.9 mm, preferably 0.3 to 1.9 mm.

Furthermore, the monolithic metal tube may be defined by its length. The "length" of the monolithic metal tube is also referred to herein as the "axial dimension" of the monolithic metal tube. According to one embodiment, the monolithic metal tube has a length in the range of 0.3 to 5 mm, preferably 1.0 to 5.0 mm, most preferably in the range of 1.0 to 4.0 mm. According to one preferred embodiment, the monolithic metal tube has an outer diameter in the range of 0.2 to 3.0 mm, an inner diameter in the range of 0.1 to 2.9 mm, and a length in the range of 0.3 to 5 mm, preferably 1.0 to 5.0 mm. According to one preferred embodiment, the monolithic metal tube has an outer diameter in the range of 0.5 to 2.0 mm, an inner diameter in the range of 0.3 to 1.9 mm, and a length in the range of 1.0 to 4.0 mm.

The monolithic metal tube may be further specified by its wall thickness. According to one embodiment, the wall of the monolithic metal tube has a thickness in at least one section of the wall in the range of 0.05 to 0.4 mm, preferably 0.075 to 0.3 mm. Preferably, the at least one section of the wall having a thickness in the range of 0.05 to 0.4 mm, preferably 0.075 to 0.3 mm, does not contain the contacting channel.

According to a preferred embodiment, the monolithic metal tube is a metal selected from the group consisting of iron, silver, copper, nickel, palladium, platinum, gold, iridium, steel, titanium, hafnium, niobium, tantalum, cobalt, chromium, zirconium, rhenium, tungsten, molybdenum, and alloys of each one of these metals, preferably from the group consisting of nickel cobalt alloy, stainless steel, platinum and platinum alloys, and most preferably is a platinum iridium alloy (e.g. Pt/Ir 10 or Pt/Ir 20), and wherein the monolithic metal tube has an outer diameter in the range of 0.2 to 3.0 mm, an inner diameter in the range of 0.1 to 2.9 mm, and a length in the range of 0.3 to 5 mm, preferably 1.0 to 5.0 mm.

The monolithic metal tube has a wall which in one section comprises a contacting channel.

It is to be understood that the monolithic metal tube is not limited to one contacting channel. The monolithic metal tube may comprise one or more additional contacting channels as defined herein in other sections of its wall depending on the desired use of the ring electrode. The number of contacting channels can be chosen by a skilled person based on the specification of the inventive ring electrode herein and the desired field of application. According to one embodiment, the monolithic metal tube has one contacting channel. According to another embodiment, the monolithic metal tube has two or more contacting channel (e.g. three contacting channel).

The contacting channel extends into the axial direction of the monolithic metal tube, wherein the contacting channel makes at least a partial turn around the center axis of the monolithic metal tube. This can be understood in that the contacting channel has a first end and a second end, wherein the first end is offset to the second end by a turn around the center axis of the monolithic metal tube of at least 5°.

The contacting channel can make a partial turn around the center axis of the monolithic metal tube. The contacting channel can make a turn in the range of 5 to 355° around the center axis of the monolithic metal tube. According to a preferred embodiment, the contacting channel makes a partial turn in the range of 5 to 270°, preferably in the range of 25 to 245°, more preferably in the range of 45 to 180° or 90 to 180°, around the center axis of the monolithic metal tube. For example, the contacting channel can make a partial turn of about 125° around the center axis of the monolithic metal tube as shown in e.g. Figure 2.

According to one preferred embodiment, the contacting channel has a curved shape and makes a partial turn around the center axis of the monolithic metal tube.

Alternatively, it is possible that the contacting channel makes a full turn (360°) or more around the center axis of the monolithic metal tube. In such case, the contacting channel can have a helical shape. According to one preferred embodiment, the contacting channel has a helical shape. According to one preferred embodiment, the contacting channel has a helical shape and makes a full turn or more around the center axis of the monolithic metal tube.

The contacting channel can be defined by its pitch. The "pitch" in the meaning of the present invention is the distance in the axial direction of the ring electrode, which is needed, or would be needed, by the contacting channel for making a full 360° turn around the center axis of the ring electrode.

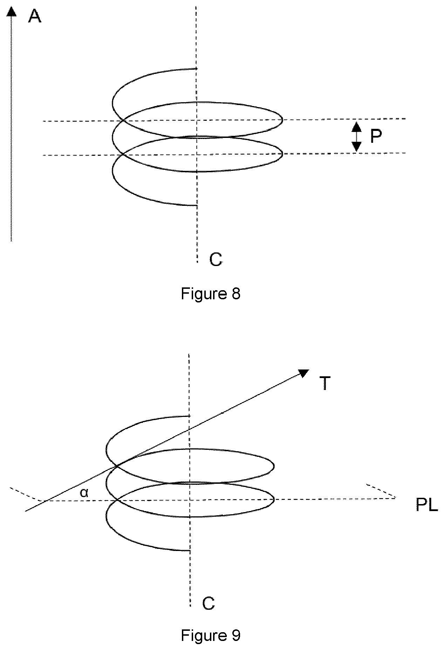

For illustrative purposes, the pitch of a helix is shown in Figure 8 by the distance P in the axial direction A, which is needed by the curve to make a full 360° helical turn around the center axis C.

The pitch of the contacting channel can be determined be measuring the respective distance in the axial direction of the contacting channel on the ring electrode. In case the contacting channel does not make a full 360° turn, the contacting channel of the ring electrode can be extrapolated to a full 360° turn of the contacting channel. "Extrapolating" means in this context that the entire dimensions of the contacting channel are multiplied in the axial direction of the electrode until a 360° turn around the center axis is completed.

According to one embodiment, the contacting channel has a pitch in the range of 0.5 to 18 mm. According to one embodiment, the contacting channel has a pitch in the range of 3 to 18 mm. According to one embodiment, the contacting channel has a pitch in the range of 6 to 12 mm. For example, the contacting channel can have a pitch of about 9 mm.

According to one embodiment, the contacting channel has a helical shape, and a pitch in the range of 0.5 to 18 mm, preferably 3 to 18 mm, more preferably in the range of 6 to 12 mm.

The contacting channel can be defined by its pitch angle. The "pitch angle" in the meaning of the present invention is the angle between a tangent to the center of the contacting channel and a plane perpendicular to the center axis of the ring electrode. For illustrative purposes, the pitch angle α of a helix is illustrated in Figure 9 by the angle between a tangent T to the helix and a plane PL perpendicular to the center axis C of the helix.

The pitch angle of the contacting channel may be constant or may be different at different positions along the length of the contacting channel.

According to one preferred embodiment, the contacting channel has a pitch angle in the range of 5 to 85° preferably in the range of 30 to 85°, more preferably in the range of 45 to 85°. According to one preferred embodiment, the contacting channel has a constant pitch angle in the range of 5 to 85°, preferably in the range of 30 to 85°. According to another preferred embodiment, the contacting channel has a pitch angle in the range of 5 to 85°, preferably in the range of 30 to 85°, and wherein the pitch angle is different at different positions along the length of the contacting channel.

It is to be understood that the shape of the contacting channel as defined herein is preferably matched with the shape of a wire which is to be contacted to the electrode via the contacting channel. In this way the stress applied to the wire contacted to the electrode can be minimized.

The pitch of the contacting channel can be matched with the pitch of a wire which is to be contacted to the electrode. For example, if a wire to be contacted has a high pitch, the contacting channel can be adapted to have a high pitch.

The pitch angle of the contacting channel can be matched with the pitch angle of a wire which is to be contacted to the electrode. For example, if a wire to be contacted has a low pitch angle, the contacting channel may be designed with a low pitch angle.

The contacting channel has an inner diameter. The inner diameter is not specifically limited as long as it allows for inserting a wire into the contacting channel. According to one embodiment, the contacting channel has an inner diameter in the range of 0.05 to 2.0 mm, preferably in the range of 0.1 to 2.0 mm, more preferably in the range of 0.1 to 1.0 mm.

The inner diameter of the contacting channel can be constant or the inner diameter can vary along the length of the contacting channel. For example, it is possible to provide the ring electrode with a contacting channel, wherein the inner diameter decreases from the first end to the second end of the contacting channel. This structure has the advantage that a wire can be easily fitted into the first end of the contacting channel, while still providing a reliable contact (e.g. by laser welding) between wire and electrode in the narrower part of the contacting channel (e.g. at the second end).

The contacting channel can have a specific length which is defined herein in relation to the total length of the monolithic metal tube. The "length" of the contacting channel is defined by the distance along the center axis of the ring electrode from a first radial plane of the electrode, which comprises the first end of the channel, to a second radial plane of the electrode, which comprises the second end of the channel.

According to one embodiment, the contacting channel has a length in the range of 20 to 100%, preferably in the range of 50 to 100%, more preferably in the range of 75 to 100%, of the total length of monolithic metal tube.

Hence, it is possible that the contacting channel has a length of 100% of the total length of the monolithic metal tube. This is one preferred embodiment. In this embodiment, the first end of the contacting channel is at a first front face of the monolithic metal tube and the second end of the contacting channel is at a second front face of the monolithic metal tube. This embodiment is illustrated e.g. in Figure 1 of the present application.

However, it is also possible, and sometimes preferred, that the contacting channel has a length which is below 100% of the total length of the monolithic metal tube. For example, the contacting channel can have a length in the range of 20 to 80% of the total length of the contacting channel.

The position of the contacting channel in the wall of the monolithic metal tube is not specifically limited as long as the contacting channel is suitable for contacting a wire.

The contacting channel may be fully included into the one section of the wall of the monolithic metal tube. "Fully included" means that the inner surface of the monolithic metal tube has a cylindrical shape, and that the one wall section of the monolithic metal tube which comprises the contacting channel does not protrude into the inner cavity of the monolithic metal tube. According to one embodiment, the contacting channel is fully included into the wall of the monolithic metal tube.

Whether the contacting channel can be fully included into the wall of the monolithic metal tube, can depend on the total thickness of the wall of the monolithic metal tube. For example, a monolithic metal tube having a thick wall (e.g. in the range of 0.3 to 1.0 mm) may fully include the contacting channel into one section of its wall.

It is however preferred that the one section of the wall, which comprises the contacting channel, protrudes into the inner cavity of the monolithic metal tube. According to one embodiment, the section of the wall, which comprises the contacting channel, protrudes into an inner cavity of the monolithic metal tube. This embodiment is illustrated in e.g. Figure 1 of the present application.

The wall of the monolithic metal tube can be subdivided into (1) one or more wall sections which comprise the one or more contacting channel and (2) one or more wall sections which do not contain a contacting channel. The monolithic metal tube can have different wall thicknesses in different sections. According to one embodiment, the one section of the wall which comprises the contacting channel has a thickness in the range of 0.2 to 1.0 mm, and another section of the wall which does not comprise the contacting channel has a thickness in the range of 0.05 to 0.4 mm, preferably 0.075 to 0.3 mm.

Furthermore, the distance between the center of the contacting channel and the outer lateral surface of the monolithic metal tube can decrease from a first end to a second end of the contacting channel. In other words, it is possible that the contacting channel gradually moves in radial direction towards the outer lateral surface of the monolithic metal tube. In such case, it is possible to insert a wire into the first end of a contacting channel and contact the wire to the electrode in the proximity of the second end by e.g. a laser welding step. For certain contacting processes such as laser welding, it can be beneficial that the outer lateral surface of the monolithic metal tube is in close proximity to the contacting channel.

According to one embodiment, the distance between the center of the contacting channel and the outer lateral surface of the monolithic metal tube can decrease from a first end to a second end of the contacting channel. Preferably, the distance between the center of the contacting channel and the outer lateral surface of the monolithic metal tube decreases from a first end to a second end of the contacting channel by a value in the range of 5 to 50%.

The outer lateral surface of the monolithic metal tube may be provided with a specific functionality.

For example, the outer lateral surface of the monolithic metal tube may have an opening or a detection mark which helps to create a reliable contact between a wire and the electrode by a contacting step such as a welding step (preferably a laser welding step).

According to one embodiment, the outer lateral surface of the monolithic metal tube has an opening which connects the outside to the contacting channel. Preferably, the opening is a weld slot for contacting a wire to the electrode by a welding step (e.g. laser welding). According to one preferred embodiment, the opening has a length in the range of 0.1 to 1.0 mm, preferably 0.2 to 0.6 mm, and/or a width in the range of 0.01 to 0.1 mm, preferably, 0.02 to 0.05 mm. According to one preferred embodiment, the opening has a length in the range of 0.1 to 1.0 mm, preferably 0.2 to 0.6 mm, and a width in the range of 0.01 to 0.1 mm, preferably, 0.02 to 0.05 mm.

According to one embodiment, the outer lateral surface of the monolithic metal tube has a detection mark. A "detection mark" in the meaning of the present invention is a structural modification of the outer lateral surface of the monolithic metal tube. The detection mark can serve as a guidance for a welding step (e.g. laser welding). For example, the detection mark can indicate where the contacting channel and/or a wire is located or where the wall between the outside and the contacting channel is the thinnest. Thereby, a more reliable contact between a wire and the electrode can be achieved.

Another aspect of the present invention is directed to a medical device comprising the ring electrode according to the invention and a wire, wherein the ring electrode is contacted to the wire by its contacting channel.

The ring electrode and the wire are preferably parts of a lead in the medical device.

According to one embodiment, the medical device is an implantable medical device, and preferably an active implantable medical device (AIMD). Preferred AIMDs are, for example, cardiac pacemakers, cardiac defibrillators, neurostimulators and/or neuromodulators, cochlea implants, implantable cardioverters, nerve, brain, organ or muscle stimulators as well as implantable monitoring devices, hearing aids, retinal implants, muscle stimulators, implantable drug pumps, artificial hearts, bone growth stimulators, prostate implants, stomach implants or the like. In one preferred embodiment, the medical device is a neuromodulator (e.g. spinal cord stimulator or a deep brain stimulator).

In one preferred embodiment, the medical device is a temporary or short-term used medical device such as a catheter.

In one preferred embodiment, the medical device is a device for use in treating cardiac arrhythmias, chronic pain and/or neurological diseases.

Another aspect of the present invention relates to a process for preparing a ring electrode according to any one of the preceding claims, wherein the process comprises the step of preparing a monolithic metal tube by a metal additive manufacturing process.

The present invention is further based on the finding that the monolithic metal tube having the special design as described above can be prepared by a metal additive manufacturing process.

The monolithic metal tube has a wall which in one section comprises a contacting channel, wherein the contacting channel extends into the axial direction of the monolithic metal tube, and wherein the contacting channel makes at least a partial turn around the center axis of the monolithic metal tube.

The embodiments, and preferred embodiments, of the monolithic metal tube prepared by the additive manufacturing process are the same as described above in connection with the monolithic metal tube of the ring electrode.

The metal additive manufacturing process may be a selective laser melting process, a selective laser sintering process or a direct energy deposition process, and preferably is a selective laser melting process.

The metal additive manufacturing process is preferably a selective laser melting (SLM) process. SLM processes are known in the art. The SLM process may proceed as a powder bed fusion process that uses high intensity laser as an energy source to melt and fuse selective regions of metal powder, layer by layer, according to a computer aided design (CAD) data. After CAD data preparation and processing, the SLM process may start with laying a layer of metal powder in a building chamber. After the powder is laid, a high energy density laser may be used to melt and fuse the area of the metal powder. Once the laser scanning is completed, the building platform may be lowered, a next layer of powder may be deposited on top and the laser may scan a new layer. The process may then be repeated for successive layers of powder until the desired monolithic metal tube is finished. A suitable device for carrying out the inventive process is a Realizer SLM-50.

The metal powder may have any particle size that is suitable for preparing a ring electrode according to the invention. For example, the metal powder may have a particle size d₅₀ in the range of 5 to 15 µm. A suitable metal powder is, for example, a Pt/lr 10 gas-spray aeration powder having a particle size d₅₀ in the range of 5 to 15 µm. Particle size can be determined by laser diffraction as known in the art.

According to one preferred embodiment, the process comprises the steps of:
a) preparing a monolithic metal tube by metal additive manufacturing, and
b) post-processing the monolithic metal tube obtained in step a) to obtain the ring electrode according to the present invention.

For example, post-processing of the monolithic metal tube in step b) may comprise one or more steps from the group of cleaning, polishing, surface structuring (e.g. laser structuring) and coating of the monolithic metal tube.

### EXAMPLES

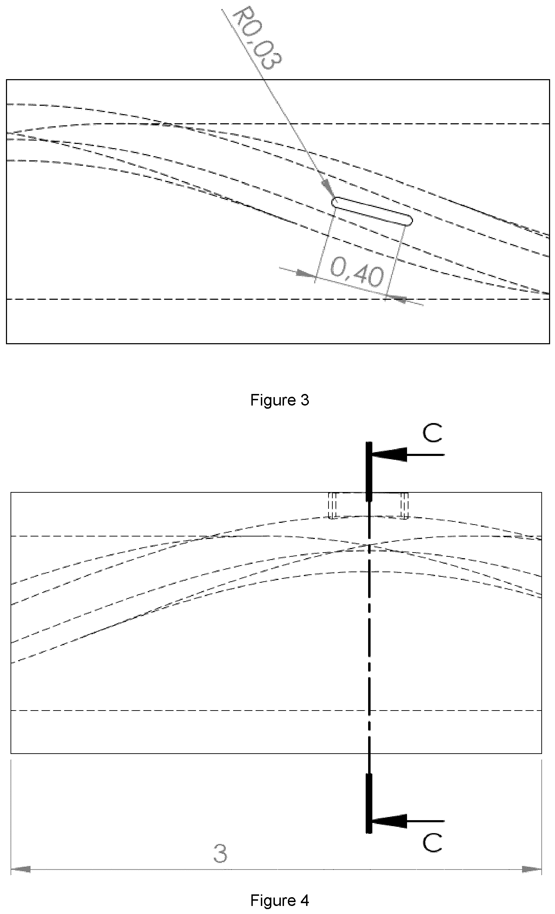

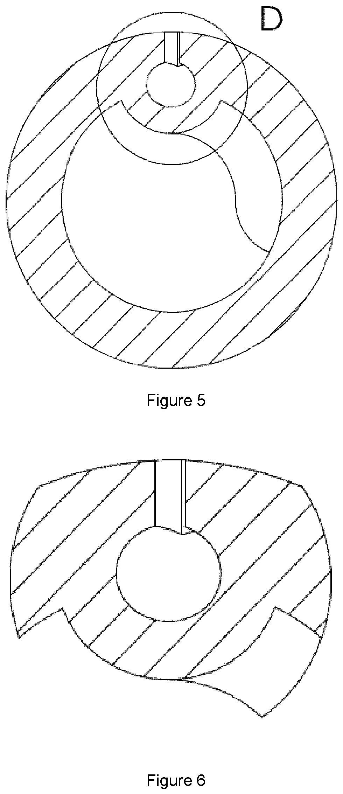

Two ring electrodes were prepared on the basis of the technical drawings as shown in Figures 2 to 6.

The ring electrodes were prepared by metal additive manufacturing (selective laser melting) on a *Realizer SLM-50* device using a Pt/lr 10 gas-spray aeration powder with a particle size of 5 to 15 µm.

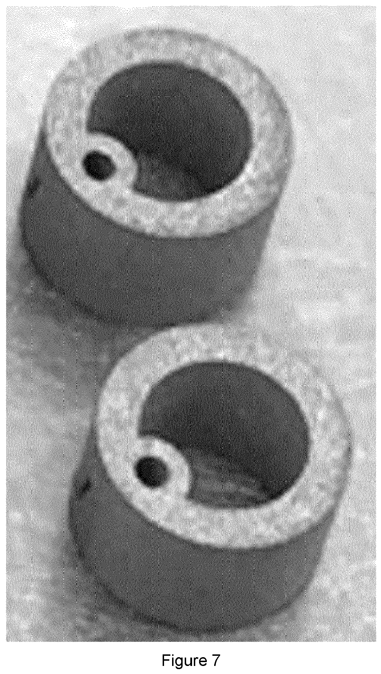

The ring electrodes had a length of 3 mm, an outer diameter of 1.50 mm and a maximum inner diameter of 1 mm. The opening on the outer lateral surface of the ring electrodes had a length of 0.40 mm and a width of 0.03 mm. The opening connects the outside to the contacting channel of the electrode. An image of the inventive ring electrodes is shown in Figure 7.

The features disclosed in the claims, the specification, and the drawings maybe essential for different embodiments of the claimed invention, both separately and in any combination with each other.

## Claims

1. A ring electrode (1) for a medical device comprising a monolithic metal tube (2),
wherein the monolithic metal tube (2) has a wall which in one section comprises a contacting channel (3),
wherein the contacting channel (3) extends into the axial direction of the monolithic metal tube (2), and wherein the contacting channel (3) makes at least a partial turn around the center axis of the monolithic metal tube.

2. The ring electrode according to claim 1, wherein the contacting channel (3) makes a partial turn in the range of 5 to 270°, preferably in the range of 25 to 245°, around the center axis of the monolithic metal tube (2), or
wherein the contacting channel (3) makes a complete turn or more around the center axis of the monolithic metal tube (2).

3. The ring electrode according to claim 1 or 2, wherein the contacting channel (3) has a pitch in the range of 0.5 to 18 mm,
wherein the pitch is the distance in the axial direction of the ring electrode, which is needed, or would be needed, by the contacting channel for making a full 360° turn around the center axis of the ring electrode.

4. The ring electrode according to any one of the preceding claims, wherein the contacting channel (3) has a pitch angle in the range of 5 to 85°, preferably 30 to 85°,
wherein the pitch angle is the angle between a tangent to the center of the contacting channel and a plane perpendicular to the center axis of the ring electrode.

5. The ring electrode according to any one of the preceding claims, wherein the section of the wall, which comprises the contacting channel (3), protrudes into an inner cavity of the monolithic metal tube (2).

6. The ring electrode according to any one of the preceding claims, wherein the contacting channel (3) has an inner diameter in the range of 0.05 to 2.0 mm.

7. The ring electrode according to any one of the preceding claims, wherein the contacting channel (3) has a length in the range of 20 to 100%, of the total length of monolithic metal tube (2).

8. The ring electrode according to any one of the preceding claims, wherein the monolithic metal tube (2) has an outer diameter in the range of 0.2 to 3.0 mm, and an inner diameter in the range of 0.1 to 2.9 mm.

9. The ring electrode according to any one of the preceding claims, wherein the monolithic metal tube (2) has a length in the range of 0.3 to 5 mm, preferably 1.0 to 5.0 mm.

10. The ring electrode according to any one of the preceding claims, wherein the wall of the monolithic metal (2) tube has a thickness in at least one section of the wall in the range of 0.05 to 0.4 mm.

11. The ring electrode according to any one of the preceding claims, wherein the outer lateral surface of the monolithic metal tube has an opening (4) which connects the outside to the contacting channel.

12. The ring electrode according to any one of the preceding claims, wherein the outer lateral surface of the monolithic metal tube (2) has a detection mark.

13. The ring electrode according to any one of the preceding claims, wherein the monolithic metal tube (2) is a metal selected from the group consisting of iron, silver, copper, nickel, palladium, platinum, gold, iridium, steel, titanium, hafnium, niobium, tantalum, cobalt, chromium, zirconium, rhenium, tungsten, molybdenum, and alloys of each one of these metals,
preferably selected from the group consisting of nickel cobalt alloy, steel, platinum and platinum alloys, and
most preferably is a platinum iridium alloy.

14. A medical device comprising the ring electrode according to any one of the preceding claims 1 to 13 and a wire, wherein the ring electrode is contacted to the wire by its contacting channel.

15. A process for preparing a ring electrode according to any one of claims 1 to 13, wherein the process comprises a step of preparing the monolithic metal tube by a metal additive manufacturing process.
